# EUROPEAN PATENT APPLICATION

(11) **EP 2 022 345 A1**
(43) Date of publication of application: **11.02.2009**
(21) Application number: 07730461.6
(22) Date of filing: 13.04.2007
(51) Int. Cl.: A23L 1/30, A23L 2/52, A23C 9/152, A61K 36/752, A61K 31/7048

(54) **FOOD COMPOSITION FOR FUNCTIONAL FOODS AND NUTRITIONAL SUPPLEMENTS**

(30) Priority: 02.06.2006 ES 200601483
(71) Applicant: Furfural Espanol, S.A., 30820 Alcantarilla (ES)
(72) Inventor: BENAVENTE-GARCÍA GARCÍA, Obdulio, E-30100 Murcia (ES); CASTILLO SÁNCHEZ, Julián, E-30100 Murcia (ES); LORENTE SALINAS, Juan, E-30002 Murcia (ES)
(74) Representative: Carvajal y Urquijo, Isabel
(86) International application number: PCT/ES2007/000221
(87) International publication number: WO 2007/141351

(57) **Abstract**

A food composition for functional foods and nutritional supplements, obtained from the combination of extracts derived from citrus species, which comprises between 25 and 80% by weight of hesperidin; between 1 and 10% by weight of isonaringin; between 0.5 and 1% by weight of eriocitrin; and between 0.1 and 1% by weight of diosmin and the use of said composition in functional foods and nutritional supplements.

## Description

### Field of the Invention

The present invention relates to a food composition and use thereof in functional food products and nutritional supplements for preventing, reducing or treating cardiovascular problems such as varicose veins, "heavy leg" or "tired leg" syndrome or hemorrhoids.

### Background of the Invention

Flavonoids are a very large group of natural compounds present in almost all plants. Of all plant species, citruses are characterized by accumulating particularly high amounts of flavonoids and are an excellent source for the industrial isolation and obtaining of these compounds.

The association between flavonoid consumption and cardiovascular diseases has been studied in several epidemiological studies (Hertog et al., 1992). Flavonoid intake in the diet has been inversely associated to the risk of mortality due to cardiovascular disease and a lower risk of stroke. These effects were independent of the known risk factors for cardiovascular diseases which are high blood cholesterol levels, body mass index, hypertension, smoking, or alcohol or fat consumption. Flavonoid intake has been also associated to a lower risk of apoplexies (Hertog et al., 1993; Heli et al., 1996).

Unfortunately, modern life habits make the consumption of vegetables in our diet insufficient for covering the need that our body has for these nutrients and make it necessary to supplement our diet with daily consumption functional foods containing larger amounts of flavonoids than those that they could usually contain.

There are large population groups that have small problems derived from a bad cardiovascular function such as varicose veins, "heavy leg" or "tired leg" syndrome, hemorrhoids, etc. Flavonoids, specifically citrus flavonoids, due to their antioxidant (Benavente-Garcia et al., 1997), venotonic, anti-inflammatory (Kandaswami, 1999) and antithrombotic (Manach et al., 1999) properties, have been used in their most purified state as drugs for the treatment of pathologies derived from a bad vascular system function. However, these drugs have bioavailability problems mainly due to their low solubility in water and a high cost as the pharmaceutical products that they are.

It is therefore necessary to cover the deficiencies of a large population group with the risk of suffering from cardiovascular diseases, with the use of a composition capable of daily providing the necessary amounts of flavonoids for preventing said diseases through functional foods (dairy products, juices, beverages, snacks, bakery products, etc).

### Description of the Invention

In this sense, according to a first aspect, the present invention provides a food composition obtained from the combination of an orange extract and a lemon extract with high content of flavonoids, mainly hesperidin, isonaringin, eriocitrin and diosmin.

Orange and lemon extracts can be obtained from several methods such as extraction with solvents, such as superheated water and supercritical CO₂. A hydroalcoholic extraction on dried orange or lemon peel has been used in the present invention, which significantly improves the solubility in water of the obtained compounds and therefore the bioavailability thereof.

Combining both extracts in suitable proportions produces a product with the following flavonoid composition:

| | |
|---|---|
| Hesperidin | 25-80% |
| Isonaringin | 1-10% |
| Eriocitrin | 0.5-1% |
| Diosmin | 0.1-1% |

Different doses of the composition of the invention can be formulated in different functional foods and nutritional supplements for preventing, reducing or treating cardiovascular diseases or mild manifestations thereof, such as varicose veins, "heavy leg" or "tired leg" syndrome or hemorrhoids.

An optional embodiment of the composition comprises preferably between 70 and 80% by weight of hesperidin; between 1 and 3% by weight of isonaringin; between 0.5 and 1% by weight of eriocitrin and between 0.1 and 1% by weight of diosmin.

Suitable formulations of the present composition for its inclusion in functional food include liquid preparations such as solutions in glycerin-water or lecithin-water mixtures, or solid preparations such as micronization or microencapsulation.

Recommended doses for supplementing the diet are between 500-1000 mg/day, which is equivalent to about 250-500 mg/serving.

According to a second aspect of the invention, the present invention relates to the use of the composition in which it is added to functional food products such as milk, fermented dairy products, carbonated beverages, juices and nectars, and bakery products.

The healthy effects of the composition developed herein are described in the following example.

### Embodiment

A hydroalcoholic extraction is carried out on dried orange and lemon peel in order to obtain orange and lemon extracts is performed. Both extracts are combined to obtain a product comprising 75% of hesperidin, 2.5% of isonaringin, 0.8% of eriocitrin and 0.3% of diosmin.

The effect of the daily intake of the composition of the invention on chronic venous insufficiency (CVI) and hemorrhoid symptoms in volunteers is described

### Edema in legs

Study on 20 volunteers treated with 1000 mg/day of the composition of the invention (hereinafter "composition") for 2 months.

| | % edema volume reduction |
|---|---|
| Control | 0 |
| Placebo | 3.5 |
| Composition | 13 |

### Ulcer healing

Study on 15 volunteers treated with 1000 mg/day of the composition for 6 months.

| | % Complete ulcer healing |
|---|---|
| Control | 22.4 |
| Placebo | 27.5 |
| Composition | 55.5 |

The control treatment corresponds to the traditional therapy. Both the placebo and the composition of the invention were administered in the same dose together with the traditional therapy.

### CVI Symptoms

A study on 35 volunteers treated with 1000 mg/day of the composition of the invention for 12 months. The main evaluated symptoms are: leg heaviness, leg swelling, pain and cramps in the legs.

| Symptom | % Reduction in relation to the non-treated control |
|---|---|
| Heaviness | 52 |
| Swelling | 57 |
| Cramps | 76 |

### Hemorrhoids

Study on 10 volunteers treated with 1000 mg/day of the composition for 1 month. The evaluated symptoms were hemorrhage and inflammation.

| Weeks % Reduction of the symptoms | |
|---|---|
| 1 | 90 |
| 4 | 100 |

The results clearly show that the intake of the present composition significantly improves the CVI and hemorrhoid symptoms, improving the life quality of the treated volunteers.

## Claims

1. A food composition for functional foods and nutritional supplements, obtained from the combination of extracts derived from citrus species, **characterized in that** it comprises the following flavonoids: between 25 and 80% by weight of hesperidin; between 1 and 10% by weight of isonaringin; between 0.5 and 1% by weight of eriocitrin; and between 0.1 and 1% by weight of diosmin.

2. The food composition according to claim 1, **characterized in that** it comprises preferably between 70 and 80% by weight of hesperidin.

3. The food composition according to claim 1, **characterized in that** it comprises preferably between 1 and 3% by weight of isonaringin.

4. The food composition according to claim 1, **characterized in that** it comprises preferably between 0.5 and 1 % by weight of eriocitrin.

5. The food composition according to claim 1, **characterized in that** it comprises preferably between 0.5 and 1% by weight of diosmin.

6. Use of the composition according to claim 1, **characterized in that** it is added to functional food products and nutritional supplements, such as milk, fermented dairy products, carbonated beverages, juices and nectars, bakery products.
